# EUROPEAN PATENT SPECIFICATION

(11) **EP 1 937 232 B1**
(45) Date of publication and mention of the grant of the patent: **21.12.2011**
(21) Application number: 06806182.9
(22) Date of filing: 11.10.2006
(51) Int. Cl.: A61K 31/05, A61K 31/353, A61P 21/00

(54) **NUTRACEUTICAL COMPOSITION FOR THE TREATMENT OF MUSCLE WASTING**
NUTRAZEUTISCHE ZUSAMMENSETZUNGEN ZUR BEHANDLUNG VON MUSKELSCHWUND
COMPOSITIONS NUTRACEUTIQUES POUR LE TRAITEMENT DE L'ATROPHIE MUSCULAIRE

(30) Priority: 14.10.2005 EP 05022414; 15.05.2006 EP 06009943
(43) Date of publication of application: 02.07.2008
(73) Proprietor: DSM IP Assets B.V., 6411 TE Heerlen (NL)
(72) Inventor: RAEDERSTORFF, Daniel, F-68720 Flaxlanden (FR); WANG-SCHMIDT, Ying, CH-8143 Stallikon (CH); WOLFRAM, Swen, 79761 Waldshut-Tiengen (DE)
(74) Representative: Steck, Melanie
(86) International application number: PCT/EP2006/009814
(87) International publication number: WO 2007/042271

(56) References cited:
- WO-A-2004/105517
- WO-A-2007/006135
- BUETLER TIMO M ET AL: "Green tea extract decreases muscle necrosis in mdx mice and protects against reactive oxygen species" AMERICAN JOURNAL OF CLINICAL NUTRITION, vol. 75, no. 4, April 2002 (2002-04), pages 749-753, XP002459513 ISSN: 0002-9165
- WYKE S M ET AL: "Induction of proteasome expression in skeletal muscle is attenuated by inhibitors of NF-kappaB activation" BRITISH JOURNAL OF CANCER, LONDON, GB, vol. 91, no. 9, 1 November 2004 (2004-11-01), pages 1742-1750, XP002445876 ISSN: 0007-0920
- MATSUMOTO K ET AL: "Effect of apple polyphenol on muscle atrophy and subsequent recovery in immobilized rats" MEDICINE AND SCIENCE IN SPORTS AND EXERCISE, vol. 33, no. 5 Supplement, May 2001 (2001-05), page S163, XP009092403 & 48TH ANNUAL MEETING OF THE AMERICAN COLLEGE OF SPORTS MEDICINE; BALTIMORE, MARYLAND, USA; MAY 30-JUNE 02, 2001 ISSN: 0195-9131
- HAMADA K ET AL: "Effect of apple polyphenol on oxidative stress during recovery from muscle atrophy" MEDICINE AND SCIENCE IN SPORTS AND EXERCISE, vol. 33, no. 5 Supplement, May 2001 (2001-05), page S217, XP009092404 & 48TH ANNUAL MEETING OF THE AMERICAN COLLEGE OF SPORTS MEDICINE; BALTIMORE, MARYLAND, USA; MAY 30-JUNE 02, 2001 ISSN: 0195-9131
- BUCK MARTINA ET AL: "Muscle wasting and dedifferentiation induced by oxidative stress in a murine model of cachexia is prevented by inhibitors of nitric oxide synthesis and antioxidants" EMBO (EUROPEAN MOLECULAR BIOLOGY ORGANIZATION) JOURNAL, vol. 15, no. 8, 1996, pages 1753-1765, XP001537737 ISSN: 0261-4189
- DORCHIES OLIVIER M ET AL: "Green tea extract and its major polyphenol (-)-epigallocatechin gallate improve muscle function in a mouse model for Duchenne muscular dystrophy" AMERICAN JOURNAL OF PHYSIOLOGY - CELL PHYSIOLOGY, vol. 290, no. 2, February 2006 (2006-02), pages C616-C625, XP002459514 ISSN: 0363-6143

## Description

The present invention relates to a novel use of nutraceutical compositions comprising as active ingredient EGCG and resveratrol.

The compositions of the present invention are particularly intended for prevention and treatment of muscle wasting leading to muscle loss, atrophy and other associated muscle disorders in animals, in particular in mammals including humans.

The term "nutraceutical" as used herein denotes usefulness in both the nutritional and pharmaceutical field of application. Thus, the novel nutraceutical compositions can find use as supplements to food and beverages, dietary supplements and as pharmaceutical formulations for enteral or parenteral application which may be solid formulations such as capsules or tablets, or liquid formulations, such as solutions or suspensions. As will be evident from the foregoing, the term nutraceutical composition also comprises food and beverages containing the above-specified active ingredients.

The term "EGCG" is used for (-)-epigallocatechin gallate. EGCG is the major catechin found in green tea. The beneficial health effects of green tea have been mainly attributed to the catechins. In mice, tea catechins reduced diet-induced weight gain, visceral fat mass, as well as plasma leptin, triglyceride and glucose levels. Tea catechins are also known to increase energy expenditure in rats. In humans, tea catechins have been shown to reduce body weight, visceral fat mass and plasma cholesterol, insulin and glucose levels. Green tea extract was shown to significantly increase energy expenditure and fat oxidation in healthy men. Furthermore, it was shown in brown adipose tissue of rats that EGCG stimulates metabolic activity and oxygen consumption. Additionally, several animal studies demonstrated that catechins inhibited cholesterol absorption and lowered plasma cholesterol levels. In turn, epicatechins increase the fecal excretion of cholesterol and total lipids. Therefore, EGCG has an antiobesity effect, through a stimulation of thermogenesis and/or an altered fat absorption.

The effect of dietary green tea extract supplementation to protect the EDL (elongator digitorum longus) muscle in the *mdx* mouse dystrophy model is disclosed in Buetler et al., Am. J. Clin. Nutr. 75:749, 2002.

The usefulness of resveratrol for the treatment of muscle wasting in cancer cachexia is suggested in Wyke et al., Br. J. Cancer 91:1742, 2004.

Hydroxytyrosol is in the form of a single compound or of a purified plant extract, especially an olive extract. Hydroxytyrosol is the main polyphenol found in olives. Hydroxytyrosol is believed to be the antioxidant with the highest free radical scavenging capacity: double that of quercetin and more than 3 times that of epicatechin. The wastewaters generated during olive processing contain high levels of hydroxytyrosol, from which hydroxytyrosol can be recovered to produce hydroxytyrosol extracts. Hydroxytyrosol has the same health promoting properties than other polyphenols: prevention of atherosclerosis, promotion of intestinal and respiratory health and prevention of cancer. Hydroxytyrosol also reduces the oxidative stress caused by smoking.

Muscle wasting is characterized by a progressive loss of muscle mass, weakening and degeneration of muscles especially the skeletal or voluntary muscles and the cardiac muscles.

The processes by which atrophy and hypertrophy occur are conserved across mammalian species. Multiple studies have demonstrated that the same basic molecular, cellular, and physiological processes occur during atrophy in both rodents and humans. Thus, rodent models of skeletal muscle atrophy have been successfully utilized to understand and predict human atrophy responses.

Muscle wasting is due to a variety of causes and is associated with various pathologies, diseases and illnesses. These includes but are not limited to muscular dystrophies caused by genetic disorders such as Duchenne's muscular dystrophy, progressive muscular dystrophy, Becker's type muscular dystrophy, Dejerine-Landouzy muscular dystrophy, Erb's muscular dystrophy, and infantile neuroaxonal muscular dystrophy. Muscles wasting also arise from chronic diseases and age. As the body ages, an increasing proportion of skeletal muscle is replaced by fibrous tissue. Therefore, normal aging in humans is associated with progressive decrease in skeletal muscle mass and strength, a condition called sarcopenia, which contributes to frailty and falls.

Moreover, age related disorders such as hypertension, glucose intolerance and diabetes, obesity, dyslipidemia, atherosclerotic and cardiovascular disease are also associated with loss of muscle mass.

In addition other conditions such as cancer, autoimmune diseases, infectious diseases, HIV infection, AIDS, chronic inflammation, arthritis, malnutrition, renal diseases, chronic obstructive pulmonary disease (COPD), emphysema, osteomalacia, chronic lower back pain, peripheral nerve damage, spinal cord damage, chemical damage, central nervous system (CNS) damage are linked to or can cause muscle wasting. Finally, conditions resulting in muscle wasting may arise from disuse conditions such as long term immobilization due to illness or disability such as confinement in a wheelchair, prolonged bed rest, bone fracture or trauma. It is estimated that bed-rest after surgery causes loss of skeletal muscle mass of approximately 10% per week.

Untreated muscle wasting disorders can have serious health consequences.

The changes that occur during muscle wasting can lead to a weakened physical state resulting in poor performance of the body and detrimental health effects.

Thus, muscle atrophy can seriously limit the rehabilitation of patients from immobilizations. Muscle wasting due to chronic diseases can lead to premature loss of mobility and increase the risk of disease-related morbidity. Muscle wasting due to disuse is especially a serious problem in elderly, who may already suffer from age-related deficits in muscle function and mass such leading to permanent disability and premature death, as well as increased bone fracture rate. Despite the clinical importance of the condition few treatments exist to prevent or reverse the condition.

Now it has been surprisingly found that compositions containing as active ingredient EGCG and resveratrol is useful for the prevention and treatment of muscle wasting leading to muscle loss and atrophy and the associated muscle disorders in animals, in particular mammals including humans.

In a specific embodiment of the present invention the nutraceutical compositions in addition to the active ingredient(s) defined above contain at least one carotenoid from the group consisting of β-carotene, lutein, zeaxanthin, lycopene and β-cryptoxanthin.

Groups of animals of particular interest apart from mammals and humans in connection with the present invention are, e.g., domestic animals or pets, such as horses, camels dromedaries, dogs, cats and birds, and animals kept in zoological gardens. Domestic animals, pets and zoo animals will receive the active ingredients preferably via their food, e.g., via pet food, including their drinking water

Moreover, it has been found that the present compositions act on different critical pathways involved in the process of muscle loss. The compositions of the present invention increase lean muscle mass and muscular strength in animal models.

Therefore, the present invention provides compositions for treating muscle wasting disorders including for example muscular dystrophy, muscle wasting due to cancer, AIDS, rheumatoid arthritis, renal failure, uremia, chronic heart failure, age-related sarcopenia, prolonged bed-rest, spinal cord injury, stroke, bone fracture.

The compositions of the invention comprise a combination of EGCG and resveratrol. Moreover, a multi-vitamin and mineral supplement may be added to the nutraceutical compositions of the present invention to obtain an adequate amount of an essential nutrients, which is missing in some diets. The multi-vitamin and mineral supplement may also be useful for disease prevention and protection against nutritional losses and deficiencies due to lifestyle patterns.

The nutraceutical compositions of the present invention contain resveratrol in an amount sufficient to provide to a human adult (weighing about 70 kg) a dosage from about 0.5 mg/day to about 2000 mg/day, preferably from about 5 mg/day to about 500 mg/day. Thus, if the nutraceutical composition is a food or beverage the amount of resveratrol contained therein is suitably in the range from about 0.2 mg to about 500 mg per serving. If the nutraceutical composition is a pharmaceutical formulation such formulation may contain from about 0.5 mg to about 500 mg per solid dosage unit, e.g., per capsule or tablet, or from about 0.5 mg per daily dose to about 2000 mg per daily dose of a liquid formulation. EGCG is preferably used in a concentration so that the daily consumption by a human adult (weighing about 70 kg) is in the range of from 10 mg/day to 2000 mg/day. A food or beverage suitably contains about 2 mg to about 500 mg of EGCG per serving. If the nutraceutical composition is a pharmaceutical formulation such formulation may contain EGCG in an amount from about 5 mg to about 500 mg per dosage unit, e.g., per capsule or tablet, or from about 10 mg per daily dose to about 2000 mg per daily dose of a liquid formulation.

In addition to EGCG the compositions can contain hydroxyrosol. The amount of hydroxytyrosol in this composition may be such to provide a daily dosage from about 0.01 mg per kg body weight to about 60 mg per kg body weight of the subject to which it is to be administered. A food or beverage suitably contains about 0.3 mg per serving to about 1250 mg per serving of hydroxytyrosol. If the nutraceutical composition is a pharmaceutical formulation such formulation may contain hydroxytyrosol in an amount from about 1 mg to about 4000 mg per dosage unit, e.g., per capsule or tablet, or from about 1 mg per daily dose to about 4000 mg per daily dose of a liquid formulation.

In case of carotenoids from the group consisting of β-carotene, lutein, zeaxanthin, lycopene and β-cryptoxanthin such carotenoid is preferably used in a concentration so that the daily consumption by an animal including humans (e.g. weighing about 70 kg) is in the range of from 0.05 mg/day to 50 mg/day (corresponding to a daily dosage of about 0.0007 to about 0.7 mg/kg body weight), more preferably from 0.5 mg/day to 30 mg/day. A nutraceutical composition preferably comprises 0.05 mg to 50 mg of the carotenoid per serving. If the composition is a pharmaceutical composition such composition may preferably comprise the carotenoid in an amount from 0.5 mg to 50 mg per dosage unit, e.g., per capsule or tablet, or a liquid formulation unit.

The term "serving" as used herein denotes an amount of food or beverage normally ingested by a human adult with a meal at a time and may range, e.g., from about 100 g to about 500 g.

The active ingredients of the composition defined above have different mechanisms of action thus providing synergistic effects in preventing muscle loss and atrophy and the associated muscle disorders in mammals, in particular humans.

The following Examples illustrate the invention further.

### Example 1

The efficacy of resveratrol, EGCG as well as the combination of both compounds on muscle mass was tested in a 3 months feeding study in C57BLKS/J db/db mice (n=20/group). C57BLKS/J db/db mice suffer from severe metabolic disorder due to a defect in the leptin receptor and loss muscle mass as they age. Muscle wasting has been associated with chronic metabolic derangements. Moreover, muscle wasting induced by a variety of means in both rodents and humans results in similar changes in muscle anatomy, cross-sectional area, function, fiber type switching, contractile protein expression, and histology. In addition, several agents have been demonstrated to regulate skeletal muscle atrophy in both rodents and in humans. These agents include anabolic steroids, growth hormone, insulin-like growth factor 1, and beta adrenergic agonists. The data showed that skeletal muscle atrophy results from common mechanisms in both rodents and humans. Therefore, the rodent model can be used to evaluate the efficacy of compounds inhibiting muscle wasting.

Male db/db mice were obtained from Jackson Laboratory (Bar Harbor, ME, USA). Adult mice at the age of 8 weeks were used in the experiment. Mice were housed individually in plastic cages with bedding and allowed free access to standard rodent food and tap water. The animal rooms were controlled for temperature (24°C), humidity (55%), and light (12-h light-dark cycle). The animals were randomized into four groups. Resveratrol and EGCG were administered as feed-ad-mix. Corn cellulose (1% of diet) served as a carrier substance for resveratrol and EGCG as well as a placebo when used alone. Group 1 received placebo, group 2 received a diet containing 0.08% of resveratrol; group 3 received a diet containing 0.08% of EGCG; and group 4 received a diet containing 0.08% of resveratrol and 0.08 % of EGCG. Body weight and food intake were determined over the course of the study. Total muscle mass was determined by nuclear magnetic resonance (NMR) measurement after 3 months of treatment. There was no difference in food intake between the groups over the study period.

Body weight and muscle tissue weight for each treatment group is shown in Table 1.

**Table 1: Body weight (BW), muscle mass and change from baseline in db/db mice after 3 months of treatment.**

| | **2 months old mice** | | **5 months old mice** | | **Change from baseline** | |
|---|---|---|---|---|---|---|
| | BW (g) | Muscle (g) | BW (g) | Muscle (g) | BW (g) | Muscle (g) |
| **Control** | 30.2 | 16.0 | 30.7 | 15.7 | 0.5 | -0.3 |
| **Resveratrol 0.08%** | 30.3 | 16.0 | 34.2 | 17.0 | 3.9 | 1.0 |
| **EGCG 0.08%** | 30.4 | 15.7 | 31.4 | 16.2 | 1.0 | 0.5 |
| **Resveratrol 0.08% + EGCG 0.08%** | 30.4 | 16.0 | 35.7 | 17.4 | 5.3 | 1.4 |

In untreated animals (control), muscle mass is reduced by 2%. In the contrary in the resveratrol, EGCG and the resvertarol + EGCG group muscle mass is increased by 6, 3 and 9% respectively. Thus, adminstration of resveratrol, EGCG and the combination of the two prevents muscle loss in the mice. The combination of resveratrol and EGCG significantly increased muscle mass and was more potent than any of the single compound alone.

### Example 2: Pharmaceutical compositions (may be prepared by conventional formulation procedures using the ingredients specified below)

### Soft gelatin capsule (not claimed)

Soft gelatin capsules are prepared by conventional procedures using ingredients specified below:
Active ingredients: Resveratrol 10 mg and vitamin E 50 mg
Other ingredients: glycerol, water, gelatine, vegetable oil.

### Hard gelatin capsule

Hard gelatin capsules are prepared by conventional procedures using ingredients specified below:
Active ingredients: resveratrol 5 mg, EGCG 100 mg, genistein, 5 mg, vitamin E 50 mg, vitamin K 1 mg
Other ingredients: Fillers: lactose or cellulose or cellulose derivatives, Lubricant: magnesium stearate if necessary (0.5%)

### Tablet

Tablets are prepared by conventional procedures using ingredients specified below:
Active ingredients: resveratrol 5 mg, EGCG 50 mg, vitamin E 20 mg
Other ingredients: microcrystalline cellulose, silicone dioxide (SiO2), magnesium stearate, crosscarmellose sodium.

### Example 3: Food items may be prepared by conventional procedures using ingredients specified below:

A Soft Drink containing resveratrol, EGCG and hydroxytyrosol may be prepared as follows:

| *1.1 Juice concentrates and water soluble flavours* | [g] |
|---|---|
| Orange concentrate | |
| 60.3 °Brix, 5.15% acidity | 657.99 |
| Lemon concentrate | |
| 43.5 °Brix, 32.7% acidity | 95.96 |
| Orange flavour, water soluble | 3.43 |
| Apricot flavour, water soluble | 6.71 |
| Water | 26.46 |

| | |
|---|---|
| *1.2 Color* | |
| β-Carotene 10% CWS | 0.89 |
| Water | 67.65 |

| *1.3 Acid and Antioxidant* | |
|---|---|
| Ascorbic acid | 4.11 |
| Citric acid anhydrous | 0.69 |
| Water | 43.18 |
| | |

| *1.4 Stabilizers* | |
|---|---|
| Pectin | 0.20 |
| Sodium benzoate | 2.74 |
| Water | 65.60 |
| | |

| *1.5 Oil soluble flavours* | |
|---|---|
| Orange flavour, oil soluble | 0.34 |
| Orange oil distilled | 0.34 |
| | |

| *1.6 Active ingredient* | |
|---|---|
| Resveratrol, EGCG and Hydroxytyrosol in amounts providing 5 mg resveratrol / per serving, 10 mg EGCG / per serving and 5 mg hydroxytyrosol / per serving. | |

Fruit juice concentrates and water soluble flavours are mixed without incorporation of air. The color is dissolved in deionized water. Ascorbic acid and citric acid is dissolved in water. Sodium benzoate is dissolved in water. The pectin is added unter stirring and dissolved while boiling. The solution is cooled down. Orange oil and oil soluble flavours are premixed. The active ingredient as mentioned under 1.6 is stirred into the fruit juice concentrate mixture (1.1).

In order to prepare the soft drink compound all parts .1.1 to 1.6 are mixed together before homogenising using a Turrax and then a high-pressure homogenizer (p₁ = 200 bar, p₂ = 50 bar).

## Claims

1. The use of resveratrol in combination with EGCG in the manufacture of nutraceutical compositions for the prevention and treatment of muscle wasting leading to muscle loss, atrophy and other associated muscle disorders in animals in particular mammals including humans.

2. The use according to claim 1 wherein in the nutraceutical compositions in addition contain at least one carotenoid from the group consisting of β-carotene, lutein, zeaxanthin, lycopene and β-cryptoxanthin.

3. The use as in claim 1 or claim 2 wherein said resveratrol in used in an amount sufficient to provide a daily dosage of 0.03 mg per kg body weight to about 10 mg per kg body weight of the subject to which it is to be administered and said EGCG is used in an amount sufficient to provide a daily dosage of 0.1 mg per kg body weight to about 20 mg per kg body weight of the subject to which it is to be administered.

4. The use as in claim 2 wherein said carotenoid is used in an amount sufficient to provide a daily dosage of 0.0007 to 0.7 mg per kg body weight of the subject to which it is to be administered.

5. The use as in any one of claims I to 4 wherein the nutraceutical composition is a food or beverage, or a supplement composition for food or beverage.

6. The use as in any one claims 1 to 4 wherein the nutraceutical composition is a pharmaceutical composition.

## Patentansprüche

1. Verwendung von Resveratrol in Kombination mit EGCG bei der Herstellung von nutrazeutischen Zusammensetzungen für die Vorbeugung und Behandlung von Muskelschwund, der zu Muskelverlust, Atrophie und anderen damit einhergehenden Muskelerkrankungen bei Tieren, insbesondere bei Säugetieren, einschließlich dem Menschen, führt.

2. Verwendung nach Anspruch 1, wobei die nutrazeutischen Zusammensetzungen zusätzlich mindestens ein Karotinoid aus der Gruppe bestehend aus β-Karotin, Lutein, Zeaxanthin, Lycopen und β-Cryptoxanthin enthalten.

3. Verwendung nach Anspruch 1 oder Anspruch 2, wobei das Resveratrol in einer Menge verwendet wird, die ausreicht, um eine Tagesdosis von 0,03 mg pro kg Körpergewicht bis ungefähr 10 mg pro kg Körpergewicht des Patienten, an den es zu verabreichen ist, zur Verfügung zu stellen und das EGCG in einer Menge verwendet wird, die ausreicht, um eine Tagesdosis von 0,1 mg pro kg Körpergewicht bis ungefähr 20 mg pro kg Körpergewicht des Patienten, an den es zu verabreichen ist, zur Verfügung zu stellen.

4. Verwendung nach Anspruch 2, wobei das Karotinoid in einer Menge verwendet wird, die ausreicht, um eine Tagesdosis von 0,0007 bis 0,7 mg pro kg Körpergewicht des Patienten, an den es zu verabreichen ist, zur Verfügung zu stellen.

5. Verwendung nach einem der Ansprüche 1 bis 4, wobei es sich bei der nutrazeutischen Zusammensetzung um ein Nahrungsmittel oder ein Getränk oder eine Nahrungsmittel- oder Getränkzusatzmittelzusammensetzung handelt.

6. Verwendung nach einem der Ansprüche 1 bis 4, wobei es sich bei der nutrazeutischen Zusammensetzung um eine pharmazeutische Zusammensetzung handelt.

## Revendications

1. Utilisation de resvératrol en combinaison avec l'EGCG dans la fabrication de compositions nutraceutiques pour la prévention et le traitement de l'atrophie musculaire conduisant à la perte musculaire, l'atrophie et d'autres troubles musculaires associés chez les animaux, en particulier les mammifères y compris l'homme.

2. Utilisation selon la revendication 1, **caractérisée en ce que** les compositions nutraceutiques contiennent en outre au moins un caroténoïde du groupe constitué par le β-carotène, la lutéine, la zéaxanthine, le lycopène et la β-cryptoxanthine.

3. Utilisation selon la revendication 1 ou la revendication 2, **caractérisée en ce que** ledit resvératrol est utilisé dans une quantité suffisante pour donner une dose journalière de 0,03 mg par kg de poids corporel à environ 10 mg par kg de poids corporel du sujet à qui il doit être administré et ledit EGCG est utilisé dans une quantité suffisante pour donner une dose journalière de 0,1 mg par kg de poids corporel à environ 20 mg par kg de poids corporel du sujet à qui il doit être administré.

4. Utilisation selon la revendication 2, **caractérisée en ce que** ledit caroténoïde est utilisé dans une quantité suffisante pour donner une dose journalière de 0,0007 à 0,7 mg par kg de poids corporel du sujet à qui il doit être administré.

5. Utilisation selon l'une quelconque des revendications 1 à 4, **caractérisée en ce que** la composition nutraceutique est un aliment ou une boisson, ou une composition de complément pour un aliment ou une boisson.

6. Utilisation selon l'une quelconque des revendications 1 à 4, **caractérisée en ce que** la composition nutraceutique est une composition pharmaceutique.
